# EUROPEAN PATENT APPLICATION

(11) **EP 1 563 835 A1**
(43) Date of publication of application: **17.08.2005**
(21) Application number: 03775845.5
(22) Date of filing: 19.11.2003
(51) Int. Cl.: A61K 31/121, A61K 9/48, A61K 47/18, A61P 1/04

(54) **HYDROXYPROPYLMETHYLCELLULOSE CAPSULE PREPARATION HAVING TEPRENONE ENCAPSULATED THEREIN**

(30) Priority: 22.11.2002 JP 2002339970
(71) Applicant: Eisai Co., Ltd., Tokyo 112-8088 (JP)
(72) Inventor: NITTA, Katsumi, Kakamigahara-shi, Gifu 503-0861 (JP); MASAMURA, Shigeko, Gifu-shi, Gifu 500-8409 (JP)
(74) Representative: HOFFMANN - EITLE
(86) International application number: PCT/JP2003/014738
(87) International publication number: WO 2004/047822

(57) **Abstract**

The present invention provides a capsule preparation comprising a hydroxypropyl methylcellulose capsule filled with teprenone, and a capsule preparation in which the capsule is further blended with glycine, whereby delay in disintegration of the capsule preparation can be suppressed.

## Description

### Technical Field

The present invention relates to a hydroxypropyl methylcellulose (hereinafter referred to as 'HPMC') capsule preparation filled with a composition of teprenone, which is useful as a medicine for treating gastric ulcers and gastritis.

### Background Art

Teprenone is known as a medicine for treating gastric ulcers and gastritis (for example, see Patent document 1 and Patent document 2). Teprenone is an oily liquid at room temperature, and hence teprenone is adsorbed into a silicate or the like, and excipient is further added to form a powder, and then the powder is made into a preparation such as a powder, a granule, a capsule, or a tablet. Regarding the base material of capsules, a gelatin capsule having gelatin as a principal component thereof are widely used.

However, it has been found that if the gelatin capsule preparation is stored for aprolongedperiod, then the disintegration time may become longer. It is thought that this is because with a gelatin capsule preparation of teprenone, a film of the capsule is made insoluble through a reaction with peroxidation products including aldehydes produced through an oxidative decomposition reaction between the lysine residue of the gelatin and the teprenone. Although a marked lengthening of the dissolution time is not observed in dissolution tests even upon prolonged storage, and hence it cannot be said that the lengthening of the disintegration time is of an extent that product quality is affected, from the viewpoint of quality control and so on, it is nevertheless desirable to produce a capsule preparation for which changes over time are yet smaller.
[Patent document 1] Japanese Patent No. 63-44726
[Patent document 2] Japanese Patent No. 7-103019

### Disclosure of Invention

It is thus an object of the present invention to provide a capsule preparation containing teprenone for which the disintegration time does not lengthen even upon prolonged storage.

The present inventors have carried out assiduous studies to attain the above object, and as a result accomplished the present invention. A capsule preparation according to the present invention is a HPMC capsule preparation comprising a HPMC capsule filled with a teprenone-containing composition, and a HPMC capsule preparation comprising a HPMC capsule filled with a teprenone-containing composition having glycine mixed therein. Moreover, a method of suppressing delay in disintegration according to the present invention is a method of suppressing delay in disintegration of a capsule preparation in which a teprenone-containing composition having glycine mixed therein is filled into a HPMC capsule. Furthermore, a method of manufacturing a capsule preparation according to the present invention is a method of manufacturing a capsule preparation in which a teprenone-containing composition having glycine mixed therein is granulated, and then the granule is filled into an HPMC capsule.

With the HPMC capsule preparation filled with a teprenone-containing composition according to the present invention, no interaction arises between the filling and the base material of the capsule, and hence lengthening of the disintegration time of the capsule preparation in disintegration tests is not brought about, and thus a stable teprenone-containing capsule preparation can be provided. Moreover, the HPMC capsule preparation filled with a teprenone-containing composition according to the present invention exhibits good dissolution characteristics, with the dissolution behavior being similar to that of a gelatin capsule preparation. According to the present invention, an HPMC capsule preparation can thus be obtained for which the dissolution time is on a par with that of a gelatin capsule preparation, and lengthening of the disintegration time does not occur.

### BRIEF DESCRIPTION OF DRAWINGS

FIG. 1 illustrates a drawing which shows results of disintegration tests on a HPMC capsule preparation according to the present invention;
FIG. 2 illustrates a drawing which shows results of disintegration tests on a empty gelatin capsule used in the present invention; and
FIG. 3 illustrates a graph which shows results of dissolution tests on a HPMC capsule preparation according to the present invention, and on a gelatin capsule preparation.

### Best Mode for Carrying Out the Invention

A description is given below of an embodiment of the present invention. It should be noted that although the embodiment described below represents a suitable example of the present invention, there are no particular restrictions to the present invention in the description thereof, and that various changes and modifications may be made in the invention without departing from the spirit and scope of the present invention.

The teprenone used in the present invention is a compound having the undermentioned structural formula, and has the chemical name geranyl geranyl acetone, or 6,10,14,18-tetramethyl-5,9,13,17-nonadecatetraen-2-one. Teprenone theoretically has eight geometrical isomers, and all of these geometrical isomers are included in the present invention, but out of these, in the present invention it is preferable to use 3:2(5E:5Z)(9E,13Z)-6,10,14,18-tetramethyl-5,9,13,17-nonadecatetraen-2-one, which is actually used in medicines.

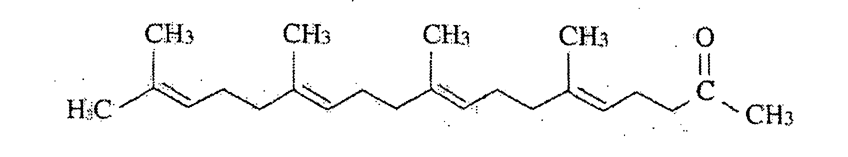

There are no particular limitations on the amount of teprenone filled into the capsules, but this amount is generally 10 mg to 200 mg, preferably 30 mg to 150 mg.

The term 'HPMC capsule' used in the present invention means a capsule having hydroxypropyl methylcellulose (HPMC) as a base material. There are no particular limitations on the HPMC capsule, but for example one made by Shionogi Qualicaps Co., Ltd. can be used. There are no particular limitations on the size of the HPMC capsule, but the total length is generally 10 mm to 23 mm, preferably 11 mm to 18 mm.

With the HPMC capsule preparation according to the present invention, glycine may be mixed into the teprenone-containing composition that is filled into the capsule. Here, glycine is an α-amino acid, being aminoacetic acid, and is an important component of various proteins. Glycine is important as a nutrient in food, and is also a permitted additive for medicines, and is easily available commercially. There are no particular limitations on the amount of glycine mixed into the teprenone-containing composition filled into the capsule, but the ratio of glycine relative to the teprenone is generally 0.001 to 0.1 parts by weight, preferably 0.01 to 0.04 parts by weight, of glycine relative to 1 part by weight of teprenone.

In addition to the teprenone-containing composition and glycine, excipient may also be added to the HPMC capsule preparation according to the present invention. As an excipient used in the present invention, it is preferable to use a sugar alcohol such as mannitol, erythritol or xylitol. The amount used of the excipient will vary depending upon the type of the excipient and so on, but is generally 5 mg to 50 mg per capsule. Furthermore, any of various antioxidants, for example vitamin E, can be added to the teprenone-containing composition in the present invention.

The capsule preparation according to the present invention can be manufactured using an ordinary method, for example as follows. That is, teprenone having vitamin E blended therein is adsorbed into a silicate, mannitol, corn starch and so on are mixed in, and then glycine that has been dissolved in Macrogol 6000 (manufactured by NOF Corporation) is added gradually, and stirring and then granulation are carried out. The granules obtained are dried, and then milling is carried out, talc or the like is mixed in, and filling into HPMC capsules is carried out, whereby the capsule preparation can be manufactured.

The present invention will be further illustrated below by means of working examples, which are given purely indicative purposes and without any limitation of the present invention.

### Working Example

3 g of tocopherol was added to 3000 g of teprenone (manufactured by Eisai Co., Ltd; ┌Selbex_{┘}), mixing was carried out thoroughly, and then the mixture was adsorbed into hydrated silicon dioxide while stirring. Next, 600 g of mannitol and 396 g of corn starch were added and mixing was carried out, and then a liquid comprising 30 g of glycine dissolved in 600 g of Macrogol 6000 (manufactured by NOF Corporation) was added gradually, and granulation was carried out. The granules obtained were dried at 70°C, milling was carried out, and then talc and 240 g of hydrated silicon dioxide were added, and the teprenone-containing granules thus obtained were filled into number 4 HPMC capsules (manufactured by Shionogi Qualicaps Co., Ltd), whereby a capsule preparation was obtained.

### Reference Example

3 g of tocopherol was added to 3000 g of teprenone, mixing was carried out thoroughly, and then the mixture was adsorbed into hydrated silicon dioxide while stirring. Next, 600 g of mannitol and 396 g of corn starch were added and mixing was carried out, and then a liquid comprising 30 g of glycine dissolved in 600 g of Macrogol 6000 (manufactured by NOF Corporation) was added gradually, and granulation was carried out. The granules obtained were dried at 70°C, milling was carried out, and then talc and 240 g of hydrated silicon dioxide were added, and the teprenone-containing granules thus obtained were filled into number 4 gelatin capsules (manufactured by Shionogi Qualicaps Co., Ltd), whereby a capsule preparation was obtained.

### HPMC Capsule Preparation Disintegration Tests

Disintegration tests were carried out using the HPMC capsule preparation according to the present invention. The HPMC capsule preparation obtained in the Working Example described above was prepared in packaging forms of Ex-1 to Ex-7 as described below. Moreover, the gelatin capsule preparation used for comparison was obtained by filling the teprenone-containing granules obtained in the Working Example into gelatin capsules (see the Reference Example).
Ex-1: 100 capsules of the capsule preparation were filled into a number 4 polypropylene bottle, and a polypropylene cap was put on.
Ex-2: Using a PTP packaging machine, the capsule preparation was subjected to PTP packaging using polypropylene film (10 capsules per sheet), and then the sheets were put into a box.
Ex-3: Using a PTP packaging machine, the capsule preparation was subjected to PTP packaging using UV-cutting type polypropylene film (10 capsules per sheet), and then the sheets were put into a box.
Ex-4: 100 capsules of the capsule preparation were filled into an aluminum bag, and heat sealing was carried out.
Ex-5: Using a PTP packaging machine, the capsule preparation was subjected to PTP packaging using polypropylene film (10 capsules per sheet).
Ex-6: Using a PTP packaging machine, the capsule preparation was subjected to PTP packaging using UV-cutting type polypropylene film (10 capsules per sheet).
Ex-7: 100 capsules of the capsule preparation were put into a glass petri dish.

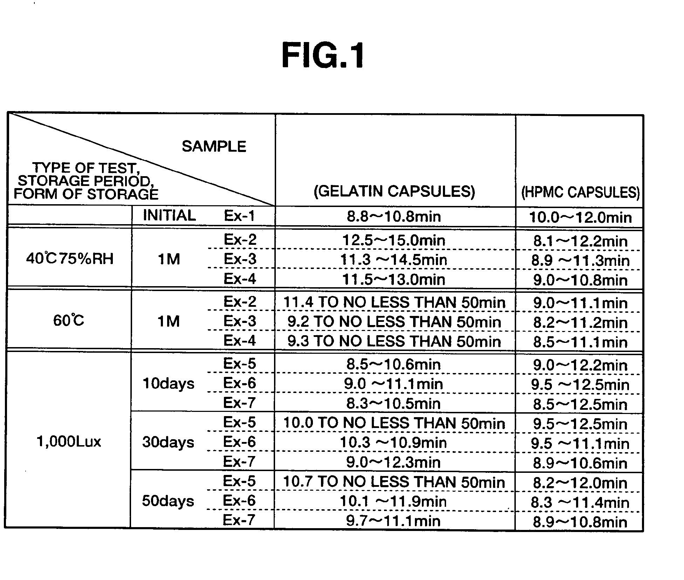

The capsule preparations of Ex-1 to Ex-7 were stored for from 10 days to a maximum of 50 days under three conditions, 60°C, 40°C with relative humidity 75%, and exposure to light at 1,000 lux, and the disintegration time was measured for each storage period. The tests were carried out in accordance with the disintegration test stipulated in the Japanese Pharmacopoeia, 14^{th} Edition. The test apparatus was adjusted such that up/down motion was carried out smoothly at 29 to 32 cycles per minute with an amplitude of 53 to 57 mm, one capsule was put into each of six auxiliary tubes used in the disintegration test for granules, each of the auxiliary tubes was put into one of the glass tubes of the test apparatus and was fixed to the bottom, and using 1,000 mL of ion exchange water as the test fluid, the test was carried out and while maintaining the test temperature at 37 ± 1 °C. The capsules in the auxiliary tubes were observed over time, and the disintegration time was taken as the time until residue including the film of capsules of the sample disappeared. The results are shown in FIG. 1.

### Empty Gelatin Capsule Disintegration Tests

For comparison, empty gelatin capsules (unfilled capsules) were stored under conditions of 40°C and relative humidity 75%, and then the disintegration test was carried out. The samples used in these disintegration tests were as follows.
Ex-8: 100 capsule preparations were filled into a number 4 polypropylene bottle.
Ex-9: 100 capsule preparations were filled into a number 4 polypropylene bottle, and a polypropylene cap was put on.

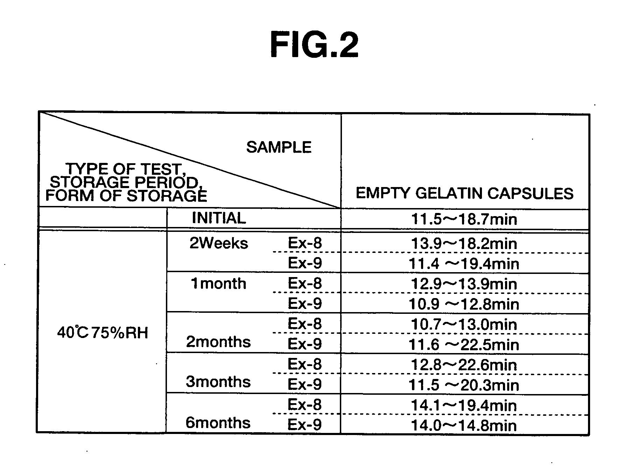

The results of the empty gelatin capsule disintegration tests are shown in FIG. 2.

As shown in FIG. 1, with the gelatin capsule preparation, it was found that under each of the conditions, the phenomenon of the gelatin film becoming insoluble over time through storage was observed, with the disintegration time lengthening, although this was not to an extent that one would expect practical use to be affected. With the empty gelatin capsules, as shown in FIG. 2, the disintegrability did not change upon storing under conditions of 40°C and relative humidity 75°C, and hence it is thought that the lengthening of the disintegration time is due to interaction between the teprenone and the gelatin capsule.

On the other hand, with the capsule preparation according to the present invention, no lengthening of the disintegration time was observed. It is thought that this is because there is no interaction between the HPMC capsule and the teprenone contained therein.

### HPMC Capsule Dissolution Tests

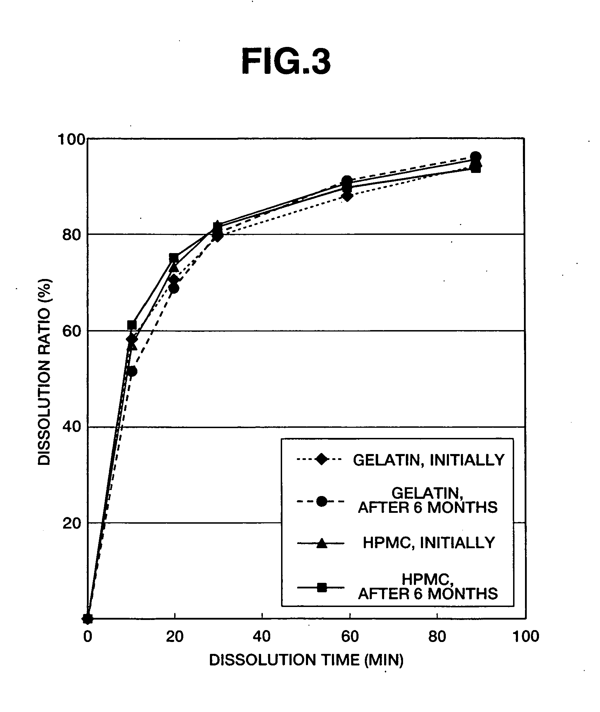

Tests comparing dissolution between the HPMC capsule preparation according to the present invention and the gelatin capsule preparation were carried out. In these tests, the HPMC capsule preparation and the gelatin capsule preparation were subjected to PTP packaging using polypropylene film (10 capsules per sheet), and then the sheets were put into a box and storage was carried out for 6 months; after that dissolution tests were carried out in accordance with the dissolution test stipulated in the Japanese Pharmacopoeia, 14^{th} Edition. FIG. 3 shows graphs comparing the dissolution times. As is clear from FIG. 3, it was found that the dissolution time for the HPMC capsule preparation according to the present invention was about the same as for the gelatin capsule preparation, both initially (before the storage) and after the storage.

### Industrial Applicability

With the HPMC capsule preparation filled with a teprenone-containing composition according to the present invention, no interaction arises between the filling and the base material of the capsule, and hence lengthening of the disintegration time of the capsule preparation in disintegration tests is not brought about, and thus a stable teprenone-containing capsule preparation can be provided. Moreover, the HPMC capsule preparation filled with a teprenone-containing composition according to the present invention exhibits good dissolution characteristics, with the dissolution behavior being similar to that of a gelatin capsule preparation. According to the present invention, a HPMC capsule preparation can thus be obtained for which the dissolution time is on a par with that of a gelatin capsule preparation, and lengthening of the disintegration time does not occur.

## Claims

1. A capsule preparation, comprising a hydroxypropyl methylcellulose capsule filled with a teprenone-containing composition.

2. A capsule preparation, comprising a hydroxypropyl methylcellulose capsule filled with a teprenone-containing composition having glycine mixed therein.

3. A method of suppressing delay in disintegration of a capsule preparation, comprising mixing glycine with a teprenone-containing composition filled into a hydroxypropyl methylcellulose capsule.

4. A method of manufacturing a capsule preparation, comprising the steps of:
granulating a teprenone-containing composition mixed with glycine; and then
filling into a hydroxypropyl methylcellulose capsule.
